# EUROPEAN PATENT APPLICATION

(11) **EP 1 978 014 A1**
(43) Date of publication of application: **08.10.2008**
(21) Application number: 07105505.7
(22) Date of filing: 02.04.2007
(51) Int. Cl.: C07C 255/41, C07C 253/30

(54) **Process for the preparation of entacapone and intermediates thereof**

(71) Applicant: ESTEVE QUIMICA, S.A., 08024 Barcelona (ES)
(72) Inventor: Bartra Sanmarti, Martí, 08015, Barcelona (ES); Solsona Rocabert, Joan Gabriel, 08225, Terrassa (Barcelona) (ES); Palomo Nicolau, Francisco, 28804, Alcalá de Henares (Madrid) (ES); Molina Ponce, Andrés, 19200, Azuqueca de Henares (Guadalajara) (ES)
(74) Representative: Barlocci, Anna

(57) **Abstract**

New preparation process for the preparation of entacapone which comprises converting the 3,4-dimethoxy-5-nitrobenzoic acid into an activated derivative which is reacted with 2-cyano-N,N-diethylacetamide in the presence of a base to give 2-cyano-3-(3,4-dimethoxy-5-nitrophenyl)-*N*,*N*-diethyl-3-hydroxyacrylamide; reacting the resulting compound with MHB(OCOR)₃ where R is (C₁-C₄)-alkyl, phenyl, or CF₃; and M is a sodium, potassium or ((C₁-C₄)-alkyl)₄N, in the presence of an appropriate solvent to give *E*-2-cyano-3-3,4-dimethoxy-5-nitrophenyl)-*N,N*-diethylacrylamide; and finally submitting the compound obtained to a demethylation reaction to yield entacapone. Both 2-cyano-3-(3,4-dimethoxy-5-nitrophenyl)-*N,N*-diethyl-3-hydroxyacrylamide and *E*-2-cyano-3-3,4-dimethoxy-5-nitrophenyl)-*N,N*-diethylacrylamide are new. Entacapone has industrial applicability as antiparkinson's agent.

## Description

The present invention relates to a process for the preparation of an active pharmaceutical ingredient known as entacapone, as well as to some new intermediates useful in such preparation process.

### BACKGROUND ART

The chemical name of entacapone is (E)-N,N-diethyl-2-cyano-3-(3,4-dihydroxy-5-nitrophenyl)acrylamide with CAS No. 130929-57-6. It was first described in GB-2200109-A by Orion Corporation. For therapeutical purposes the pure (E)-isomer is used. The product is a potent inhibitor of catechol-O-methyl-transferase enzyme (COMT) and is indicated for the treatment of Parkinson's disease.

The structure of entacapone corresponds to formula (I):

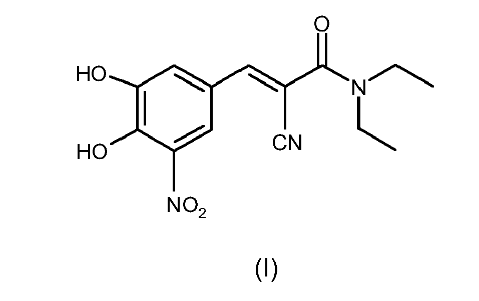

US 4963590-A describes a process for the preparation of crude N,N-diethyl-2-cyano-3-(3,4-dihydroxy-5-nitrophenyl)acrylamide by condensation of 3,4-dihydroxy-5-nitrobenzaldehyde with N,N-diethylcyanoacetamide in anhydrous ethanol and using piperidine acetate as catalyst. Although it has not been mentioned in this document, according to what has been said by the same inventors in a subsequent patent application, EP 426468-A, the resulting product is a mixture of the two geometrical isomer forms, i.e. (E)- and (Z)-.

EP 426468-A discloses the (E)- and (Z)- isomers having the following formulae:

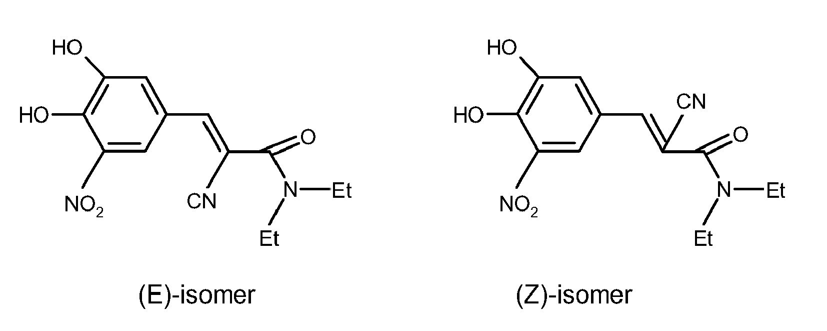

According to this document, (E)-N,N-diethyl-2-cyano-3-(3,4-dihydroxy-5-nitrophenyl)acrylamide at least may exist in two polymorphic forms, named as A and B. The (Z)-isomer as well as the polymorphic form B of the (E)-isomer have been shown to be unstable. The (Z)-isomer is transformed readily into the (E)-isomer on the influence of heat or acids. Similarly, the polymorphic form B of the (E)-isomer isomerizes slowly to the polymorphic form A on standing at room temperature. The document also describes that a crystallographically essentially pure and stable polymorphic form A may be obtained in good yield, when the crude product of entacapone is recrystallized from lower aliphatic carboxylic acids in the presence of a catalytic amount of hydrochloric or hydrobromic acid. Unfortunately, this process suffers of operation difficulty as it requires specifically designed glass reactor because of the use of corrosive material and high temperatures in highly acidic medium.

Several processes to obtain the (E)-N,N-diethyl-2-cyano-3-(3,4-dihydroxy-5-nitrophenyl) acrylamide, i.e. entacapone, has been described in the art.

Some of these processses are based on the condensation of 3,4-dihydroxy-5-nitrobenzaldehyde with N,N-diethylcyanoacetamide and differ among them in the specific reaction conditions used and/or the subsequent purification process to afford the entacapone. Thus, WO 2005/070881 describes a process for the preparation of pure entacapone by condensation of 3,4-dihydroxy-5-nitrobenzaldehyde with N,N-diethylcyanoacetamide in the presence of a base in alcoholic solution, and then isolation of the entacapone (i.e. (E)-isomer) from the crude mixture of (E)- and (Z)-isomer by extraction with ethyl acetate in acidic medium. US 2006/0258877-A describes a process for the preparation of entacapone comprising first the condensation of 3,4-dihydroxy-5-nitrobenzaldehyde with N,N-diethylcyanoacetamide in the presence of a catalyst, optionally in the presence of a phase transfer catalyst, and using ethyl acetate, acetonitrile, toluene, xylene or their mixtures as solvent, and then the treatment of the resulting isomeric mixture of (E)- and (Z)-isomer of N,N-diethyl-2-cyano-3-(3,4-dihydroxy-5-nitrophenyl)acrylamide with a catalytic amount of a halogen in a solvent to give entacapone. Both processes involve the formation of a mixture of (E)- and (Z)- isomers that must be purified in order to isolate the (E)-isomer. Furthermore, these processes use intermediates with aldehyde groups which are not stable and show colouration and purification problems.

Other processes for the preparation of entacapone are based on a demethylation reaction of a 3-alkoxy-4-hydroxy-entacapone. Thus, WO 2005/063693 describes a process for the preparation of entacapone comprising the condensation of 3-alkoxy-4-hydroxy-5-nitrobenzaldehyde with N,N-diethylcyanoacetamide in the presence of a mild acid catalyst and a solvent, to get 3-O-alkylated(methyl or ethyl) N,N-diethyl-2-cyano-3-(4-hydroxy-5-nitrophenyl)acryalamide, which is dealkylated by reaction with an acid catalyst in the presence of an organic base and solvents, and finally the resulting crude is purified to get entacapone. This process also involves the formation of a mixture of (E)- and (Z)- isomers that must be purified in order to isolate the (E)-isomer.

WO 2006/064296 describes a process for the preparation of pure entacapone based on the reaction of Vanillin with N,N-diethyl-cyanoacetamide in the presence of a weak organic acid and of an amine compound as catalyst to give (E)-N,N-diethyl-2-cyano-3-(3-methoxy-4-hydroxy-phenyl)-acrylamide, subsequently nitration to obtain (E)-N,N-diethyl-2-cyano-3-(3-methoxy-4-hydroxy-5-nitro-phenyl)-acrylamide and finally demethylation to yield entacapone. This process suffers of operation difficulty as it requires specifically designed glass reactor to carry out the nitration at industrial scale. From the teachings of the Examples, apparently the entacapone obtained suffers from colouration problems since it is recrystallized twice, each recrystallization including an activated carbon treatment to decolourate the product.

Despite the teaching of all these prior art documents, the research of new preparation processes of the entacapone is still an active field, since the industrial exploitation of known processes is difficult, as it has been pointed out in many of the above-cited documents. Thus, the provision of new preparation processes of entacapone is desirable.

### SUMMARY OF THE INVENTION

Inventors have found a new preparation process of entacapone based on the preparation of a new intermediate compound of formula (II), through a process which proceeds with a very high selectivity (E)/(Z), and on its subsequently transformation into entacapone which also proceeds with a high selectivity (E)/(Z).

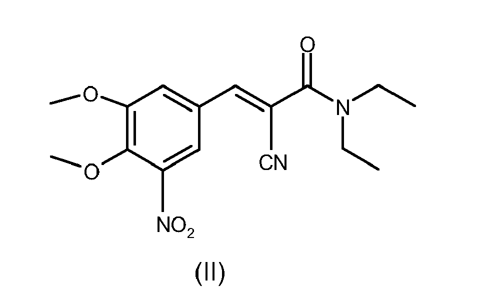

The process is specially advantageous since the intermediate compound of formula (II) not only is easily obtained in a pure form, i.e. essentially free of the (Z)-isomer and with a high chemical purity but also is stable and does not suffer from colouration problems. Likewise, the conversion of compound of formula (II) into entacapone proceeds with high yield, without formation of significant impurities, with high selectivity (E)/(Z), and the entacapone does not suffer from colouration problems. Thus, there is no need to carry out any additional purification process of the final entacapone obtained to remove the undesired (Z)-isomer or to eliminate the impurities that cause colouration which, if they are present, are very difficult to remove.

By essentially free of geometrical (Z)-isomer it is understood that the content of (Z)-isomer is equal or less than 1%. Preferably, the content of (Z)-isomer is equal or less than 0.5%. More preferably, the content of (Z)-isomer is equal or less than 0.2%.

Thus, according to an aspect of the present invention, it is provided a preparation process of entacapone of formula (I), comprising submitting the compound of formula (II) to a demethylation reaction.

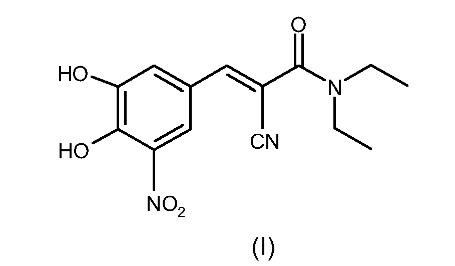

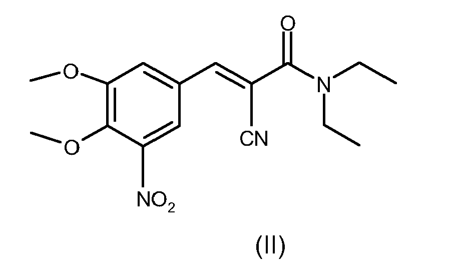

Compound of formula (II) can be previously prepared by a process comprising the following steps:
(a) reacting a compound of formula (VI) with an activating agent to give an activated derivative of the compound (VI); 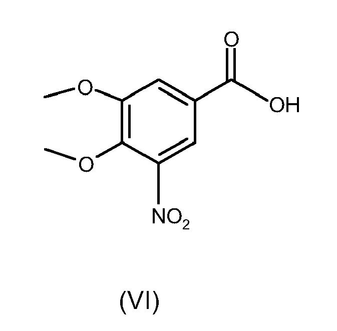
(b) reacting the activated derivative of compound of formula (VI) with a compound of formula (V) in the presence of a base, to give a compound of formula (III); and 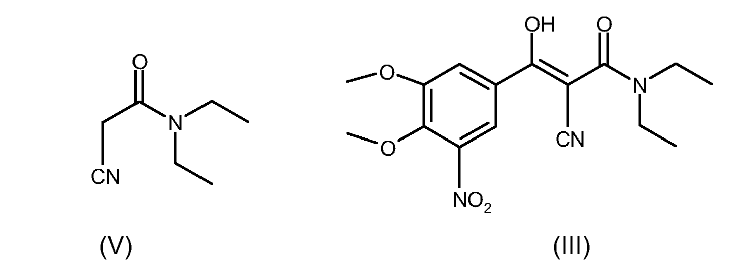
(c) reacting a compound of formula (III) with a compound of formula MHB(OCOR)₃, in the presence of an appropriate solvent to give a compound of formula (II) as described above, where R is (C₁-C₄)-alkyl, phenyl, or CF₃; and M is sodium, potassium or ((C₁-C₄)-alkyl)₄N.

The process for the preparation of compound (II) is carried out from a starting compound (VI) having both hydroxy group methylated. This structural modification provides several advantages to the process of the present invention in respect of other starting materials such as those having none or one methylated hydroxyl group. Among the advantages the following can be mentioned: colouration problems due to the presence of free hydroxyl groups are avoided; the activation of the compound (VI) takes place substantially without formation of significant impurities, for instance, the chlorination/bromination of the phenol moiety that could occur during the activation of the acid as an acyl halide is avoided; and the reaction of compound (IV) with compound (V) also takes place without formation of significant impurities, in particular the dimerization due to the formation of a fenoxy by reaction with the base is also avoided.

It is also part of the present invention the provision of single reaction steps of the global process to obtain the intermediate (II), as well as the combination of two or more sequential steps of the global process described above, the process being able to be carried out in sequential steps isolating the intermediates obtained, or alternatively, in one pot without isolation of any intermediate compounds.

Intermediate compounds of formula (II) and (III) are new. Thus, the provision of the new compounds of formula (II) and (III), which are useful intermediates for the preparation of entacapone, also forms part of the present invention.

### DETAILED DESCRIPTION OF THE INVENTION

Scheme I illustrates an embodiment of the global process for the preparation of entacapone.

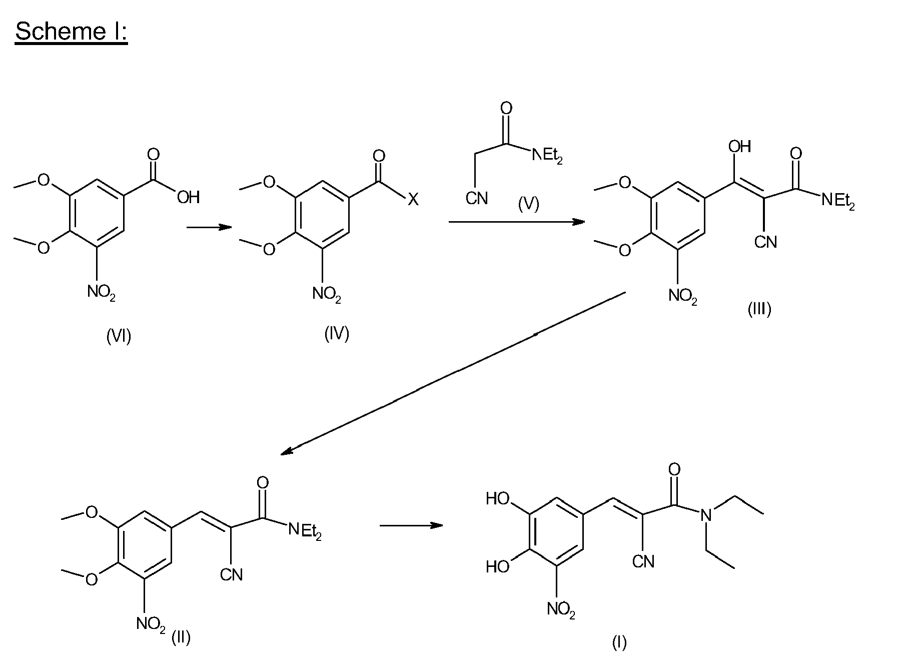

In the above Scheme compound (IV) represents an activated derivative of the compound (VI). Compound (VI) can be activated as an acyl halide such as acyl chloride or bromide; a (C₁-C₄)-alkyl ester; a carbonate, which can be obtained by using as activating agent a reagent such as CICOOR, where R₁ is a (C₁-C₄)-alkyl radical such as ethyl or isobutyl. Thus, for instance, in compound of formula (IV), X is a radical selected from CI; Br; OR₁; and OCOOR₁; where R₁ is a (C₁-C₄)-alkyl.

Other activated forms of the compound (VI) are prepared in situ in the presence of a reagent such as dicyclohexylcarbodiimide (DCC) and/or 1-hydroxybenzotriazole (HOBT), or N,N-carbonyldiimidazole (Staab reagent).

In a particular embodiment, the compound of formula (III) may be prepared by reaction of the compound of formula (VI) with a a reagent such as SOCl₂, PY₃, PY₅ where Y is CI or Br, POCl₃, or oxalyl chloride, to give a compound of formula (IV) where X is Cl or Br, followed by reaction of the compound of formula (IV) with the compound of formula (V) in the presence of a base.

Preferably, the conversion of the carboxylic compound (VI) to the acyl halide compound (IV) is carried out with an amount of acyl halide reagent comprised between 1-1.5 equivalents per equivalent of starting material. The reaction can be carried out at a temperature comprised between room temperature and the reflux temperature of the solvent employed. Preferably, the reaction temperature is comprised between 55-70 °C. Optionally, N,N-dimethylformamide can be used as catalyst, preferably in an amount comprised between 0.5-10% molar, more preferably about 5% molar, regarding to the starting material (VI). The isolation of the product of formula (IV) can be done by standard methods, for instance, the solvent can be evaporated and the crude can be used in the next step, or alternatively, it can be isolated by crystallization, precipitation, or by elimination of the solvent by evaporation followed by dispersion of the crude obtained in an appropriate solvent.

In a preferred embodiment, the reagent used for the conversion of the compound (VI) into the compound (IV) with X=Cl is SOCl₂, since the byproducts are gases and the compound of formula (IV) with X=Cl is easily isolated.

The reaction of the compound of formula (IV) with compound of formula (V) to give compound of formula (III) is carried out in the presence of a base and an aproppriate solvent. In a preferred embodiment the base is selected from sodium hydride, potassium tert-butoxide, magnesium ethoxide, and a mixture of triethylamine and magnesium chloride. Generally an excess between 2-2.5 equivalents of base per equivalent of starting compound (VI) is used. In a preferred embodiment, the reaction temperature is between about -10 °C and 10 °C. When sodium hydride is used as a base, examples of suitable solvents to carry out the reaction are tetrahydrofuran or (C₆-C₈)-aromatic hydrocarbons such as toluene or xylene. Whether a mixture of triethylamine and magnesium chloride is used as a base, generally acetonitrile is used as a solvent. The compound can be isolated as a crude to be used in the next step without purification, for instance, by acidification and evaporation of the solvent used, or alternatively it can be isolated using known methods such as extraction in a biphase system formed by water/organic solvent inmiscible in water, precipitation, and the like.

The compound of formula (II) may be prepared by reacting a compound of formula (III) with a compound of formula MHB(OCOR)₃ as defined above, such as NaHB(OAc)₃ in the presence of an appropriate solvent. Examples of suitable solvents are acetic acid, mixtures of acetic acid with ethers such as tetrahydrofuran, acetonitrile, N,N-dimethylformamide, or (C₆-C₈)-aromatic hydrocarbons such as toluene or xylene. Preferably acetic acid is used as solvent. The compound of formula (II) can be isolated for instance by precipitation with water or by extraction in a two phase system formed by water and a solvent inmiscible in water. Compound of formula MHB(OCOR)₃ can also be prepared in situ by contacting MBH₄ and RCO₂H, where R is (C₁-C₄)-alkyl, phenyl, or CF₃; and M is sodium, potassium or ((C₁-C₄)-alkyl)₄N.

Compound of formula (II) is obtained essentially free of (Z)-isomer. Optionally, It may be recrystallized to remove, for instance, mineral oil if sodium hydride has been used as base for the reaction between compound (IV) and (V). The amount of impurities due to the synthetic process are very low, including the (Z)-isomer as it has been mentioned above, therefore there is no need to carry out said recrystallization to remove such impurities, but if mineral oil is present due to the base used, then it is carried out to remove the oil.

In a particular embodiment, compound of formula (II) can be prepared starting from compound of formula (VI) in one pot, i.e. without isolation of any intermediate compound. The process includes to carry out in one pot the steps of (a) reacting a compound of formula (VI) for instance with an acyl halide reagent to obtain the compound of formula (IV) with X= CI or Br; (b) reacting the compound obtained in step (a) with a compound of formula (V) in the presence of a base to give a compound of formula (III); and finally reacting a compound of formula (III) with MHB(OCOR)₃ as defined above in the presence of an appropriate solvent to give a compound of formula (II) with the desired yield and purity.

The most adequate conditions for carrying out the different options, i.e. isolating the intermediate compounds or carrying out the process for preparing the compound of formula (II), totally or partially in one pot, vary depending on the parameters considered by the expert in the art, such as, for example, the concentration of the starting material, temperature, and the like. These can be easily determined by said skilled person in the art by routine tests and with the help of the teachings of the examples given in this description.

The starting compound (VI) of the process of the present invention is a known compound. It may be prepared from methyl 3-methoxy-4-hydroxy-5-nitrobenzoate by the process illustrated in Scheme II.

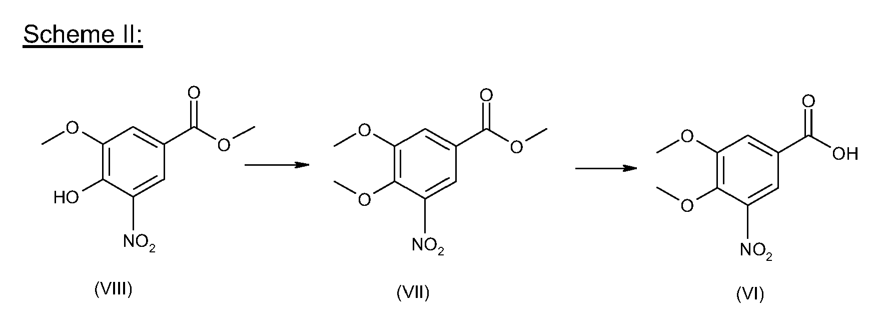

Compound (VII) can be obtained from compound (VIII) by reaction with dimethyl sulfate as metilating agent, in the presence of a base such as a carbonate or a hydroxide of an alkaline metal such as lithium, potassium or sodium, and in the presence of an appropriate solvent such as acetone, methylisobutylketone or ethyl acetate. Preferably the reaction is carried out at a temperature comprised between 40 °C and the reflux temperature of the solvent employed. The conversion of the crude compound (VII) to compound (VI) can be made by saponification using a metal alkaline hydroxide such as sodium hydroxide, or using potassium tert- butoxide. Generally, an excess of base is used. The saponification is carried out in an appropriate solvent such as an alcohol, water or mixtures thereof. Other solvents such as N,N-dimethylformamide or its mixtures with water can be used. Generally the saponification is carried out at a temperature comprised between 5-40 °C.

As it has been described above compound of formula (II) is a key intermediate of the process of the present invention for the preparation of entacapone. The process for the preparation of entacapone proceeds with high yield and purity. It comprises submitting the compound of formula (II) to a demethylation reaction. The demethylation reaction can be carried out using HBr or a Lewis acid such as AlCl₃, BBr₃, BCl₃, BF₃, Me₂BBr, or iodotrimethylsilane as demethylating agent.

In a preferred embodiment the demethylating agent is AlCl₃. Preferably, N,N-dimethylformamide is used as solvent. The demethylation reaction is preferably carried out at a temperature comprised between about 40 and 150 °C, more preferably, at a temperatute comprised between 70 and 85 °C, since at these temperatures, specially good results are achieved, mainly high conversion to the desired product with low isomerization.

Inventors have found that also specially good results in the demethylation reaction of the compound of formula (II) are obtained when AlCl₃ is used as demethylating agent in the absence of a base, in particular, in the absence of pyridine. As it is shown in the Examples (cf. Example 9 and Example 10), the yield of the reaction is meaningfully increased in the absence of a base (80% vs. 92%).

The improvement observed from the selection of the starting material and the demethylation reaction conditions is surprising since the same demethylation conditions does not work so well when the starting material is the analogous 3-alkoxy-4-hydroxy-entacapone, i.e. the intermediate compound with only a hydroxyl group methylated (cf. comparative Example 11). Known processes for the dealkylation of the analogous 3-alkoxy-4-hydroxy-entacapone use AlCl₃ and also a great excess of pyridine, and need several additional purification steps to obtain entacapone with the purity needed.

Entacapone can be isolated from the reaction medium by acidification and precipitation with water or by acidification with an alcoholic solution of an acid and precipitating the entacapone by addition of water or an alcohol. Alternatively, after acidification the entacapone can be extracted using an appropriate solvent not miscible in water. Entacapone obtained has an amount of (Z)-isomer less than 0.5%, even without carrying out any purification step during the different synthetic steps, generally less than 0.2%.

As it has been mentioned above intermediate compounds of formula (II) and (III) are new. They are stable compounds, which are obtained with high purity and with a very low amount of (Z)-isomer, the amount being lower than about 0.1 %. The crude compound (II) is a white beige solid, its direct transformation to entacapone affords a final product with the correct purity and without colouration. If the crude is recrystallized (in case that mineral oil has to be removed) the recrystallized compound (II) is a white solid.

Throughout the description and claims the word "comprise" and variations of the word, are not intended to exclude other technical features, additives, components, or steps. The disclosures in the abstract of this application is incorporated herein as reference. Additional objects, advantages and features of the invention will become apparent to those skilled in the art upon examination of the description or may be learned by practice of the invention. The following examples and drawings are provided by way of illustration, and they are not intended to be limiting of the present invention.

### EXAMPLES

### Example 1: Preparation of 3,4-dimethoxy-5-nitrobenzoic acid (compound of formula (VI))

To a suspension of methyl 4-hydroxy-3-methoxy-5-nitrobenzoate (100.0 g, 0.44 mol) in acetone (1760 mL) was added K₂CO₃ (133.8 g, 0.97 mol) and Me₂SO₄ (125.5 mL, 1.32 mol). The mixture was refluxed for 15 h, cooled to room temperature and filtered. The solvent was evaporated under reduced pressure and the residue was suspended in MeOH (1350 mL). After cooling to 10 °C, a solution of NaOH (88.1 g, 2.20 mol) in H₂O (600 mL) was added in 30 min maintaining the internal temperature below 20 °C. The mixture was stirred at 10 °C for 7 h. The resulting solution was concentrated until reaching half the initial volume, H₂O (1000 mL) was added and the rest of MeOH was eliminated. A second portion of H₂O (1000 mL) was added.

The red solution was cooled to 5 °C and 35% aqueous HCl solution was added until pH = 6.0-6.5. The resulting thick suspension was vigorously stirred and warmed to 15-20 °C. The addition of HCI was continued until pH = 1.0-1.5. After 30 min of stirring, the solid was filtered off, washed with water and dried under reduced pressure to give 92 g of the title compound (92% yield). Off-white solid. ¹H-RMN (400 MHz, CDCl₃) δ 8.09 (1 H, d, J = 1.8), 7.80 (1 H, d, J = 1.8), 4.07 (3H, s), 4.00 (3H, s). ¹³C-RMN (100.6 MHz, CDCl₃) δ 169.2, 153.9, 147.1, 144.6, 124.4, 118.5, 116.6, 62.2, 56.7.

### Example 2: Preparation of 2-cyano-3-(3,4-dimethoxy-5-nitrophenyl)-N,N-diethyl-3-hydroxvacrylamide (compound of formula (III))

Under a nitrogen atmosphere, SOCl₂ (5.78 mL, 79.2 mmol) and anhydrous DMF (0.25 mL, 3.3 mmol) were added to a suspension of 3,4-dimethoxy-5-nitrobenzoic acid (15.0 g, 66.0 mmol) in toluene (162 mL) at room temperature. The mixture was heated at 60 °C and stirred for 15 h. The organic solvent was eliminated by distillation under reduced pressure. More toluene (2 x 45 mL) was added and eliminated again. The resulting yellowish solid 3,4-dimethoxy-5-nitrobenzoyl chloride was dissolved in anhydrous THF (84 mL).

Under a nitrogen atmosphere, 60% NaH (5.28 g, 132.0 mmol) was added to a solution of 2-cyano-N,N-diethylacetamide (8.42 g, 60.1 mmol) in anhydrous THF (200 mL) at -5 °C. The resulting suspension was stirred at -5 °C for 15 min and the THF solution of 3,4-dimethoxy-5-nitrobenzoyl chloride was added over 30 min and stirred for an additional 1 h at -5 °C. The reaction mixture was heated to 0 °C, quenched by the addition of 1 N aqueous HCl solution (60 mL) and stirred for 10 min at room temperature. It was diluted with *tert-*butylmethylether (200 mL) and 1 N aqueous HCl solution (200 mL). The organic phase was separated, dried with Na₂SO₄ and concentrated in vacuo to give 24.84 g of a solid with a 83% estimated content in the title compound (quantitative yield). Orange solid. ¹H-RMN (400 MHz, CDCl₃) δ 7.89 (1 H, broad s), 7.65 (1 H, broad s), 4.04 (3H, broad s), 3.97 (3H, broad s), 3.65 (4H, broad q), 1.32 (6H, broad t). ¹³C-RMN (100.6 MHz, CDCl₃) δ 186.3, 169.9, 153.6, 145.7, 144.3, 129.6, 118.9, 117.1, 115.9, 74.2, 62.1, 56.7, 43.4, 13.4.

### Example 3: Preparation of 2-cyano-3-(3,4-dimethoxy-5-nitrophenyl)-N,N-diethyl-3-hydroxvacrylamide (compound of formula (III))

Under a nitrogen atmosphere, SOCl₂ (1.93 mL, 26.4 mmol) and DMF (0.08 mL, 1.0 mmol) were added to a suspension of 3,4-dimethoxy-5-nitrobenzoic acid (5.00 g, 22.0 mmol) in toluene (54 mL) at room temperature. The mixture was heated at 60 °C and stirred for 15 h. The organic solvent was reduced by distillation under reduced pressure to 50% of the starting volume. More toluene (2 x 25 mL) was added and eliminated to dryness. The resulting solid 3,4-dimethoxy-5-nitrobenzoyl chloride was dissolved in anhydrous ACN (20 mL).

Et₃N (7.62 mL, 55.0 mmol) was added to a suspension of 2-cyano-N,N-diethylacetamide (2.93 g, 20.9 mmol) and MgCl₂ (3.14 g, 33.0 mmol) in anhydrous ACN (50 mL) at -5 °C under a nitrogen atmosphere. The mixture was stirred at -5 °C for 30 min. The ACN solution of 3,4-dimethoxy-5-nitrobenzoyl chloride was added dropwise and the suspension was stirred for 3 h at 0 °C. The reaction mixture was quenched by the addition of 1 N HCl aqueous solution (40 mL); the phases were separated and the aqueous phase extracted with EtOAc (40 mL). The combined organic phases were washed with saturated NaCI aqueous solution (40 mL), dried with Na₂SO₄ an concentrated in vacuo to give 7.20 g (99% yield) of Z-2-cyano-3-(3,4-dimethoxy-5-nitrophenyl)-N,N-diethyl-3-hydroxyacrylamide (HPLC purity: 97.4%).

### Example 4: Preparation of E-2-cyano-3-(3,4-dimethoxy-5-nitrophenyl)-N,N-diethylacrylamide from 2-cyano-3-(3,4-dimethoxy-5-nitrophenyl)-N,N-diethyl-3-hydroxvacrylamide (compound of formula (II))

To a solution of 83% 2-cyano-3-(3,4-dimethoxy-5-nitrophenyl)-*N*,*N*-diethyl-3-hydroxyacrylamide (10.23 g, 24.3 mmol) in glacial AcOH (121 mL) was added 95% NaHB(OAc)₃ (14.63 g, 65.6 mmol) at room temperature under a nitrogen atmosphere. The mixture was stirred at 45 °C for 2.5 h, cooled to room temperature and H₂O (100 mL) was added dropwise causing the product to precipitate. The suspension was stirred at 5 °C for 1 h. The solid was filtered off, washed with cold water and dried under reduced pressure to give 6.77 g of a solid with a 88% content in *E*-2-cyano-3-(3,4-dimethoxy-5-nitrophenyl)-N,N-diethylacrylamide, 12% mineral oil of the previous step. HPLC purity: 98.9%, 0.02% (Z)-isomer (73% yield).

### Example 5: Preparation of E-2-cyano-3-(3,4-dimethoxy-5-nitrophenyl)-N,N-diethylacrylamide from 3,4-dimethoxy-5-nitrobenzoic acid in one pot (compound of formula (II))

Under a nitrogen atmosphere, SOCl₂ (3.74 mL, 51.2 mmol) and anhydrous DMF (0.16 mL, 2.1 mmol) were added to a suspension of 3,4-dimethoxy-5-nitrobenzoic acid (9.70 g, 42.7 mmol) in toluene (105 mL) at room temperature. The mixture was heated at 60 °C and stirred for 15 h. The organic solvent was reduced by distillation under reduced pressure to 50% of the starting volume. More toluene (2 x 50 mL) was added and finally eliminated to approximately 10% of the starting volume. The resulting slurry of 3,4-dimethoxy-5-nitrobenzoyl chloride was diluted with anhydrous THF (46 mL).

60% NaH (3.59 g, 89.7 mmol) was added to a solution of 2-cyano-N,N-diethylacetamide (5.69 g, 40.6 mmol) in anhydrous THF (136 mL) at -5 °C under a nitrogen atmosphere. The resulting suspension was stirred at -5 °C for 15 min and the THF solution of 3,4-dimethoxy-5-nitrobenzoyl chloride was added over 30 min and stirred for an additional 1 h at -5 °C. The reaction mixture was heated to 5 °C and quenched by the addition of glacial AcOH (30 mL). The THF was eliminated under reduced pressure and the mixture was diluted with glacial AcOH (126 mL). 95% NaHB(OAc)₃ (22.67 g, 101.6 mmol) was added at room temperature and the suspension was heated to 45 °C and stirred for 2.5 h. The solution was cooled to 30 °C and H₂O (165 mL) was added dropwise. The resulting suspension was stirred at 5 °C for 1 h. The solid was filtered off, washed with cold water and dried under reduced pressure to give 11.27 g of a solid with 13% of mineral oil and 87% content in *E*-2-cyano-3-(3,4-dimethoxy-5-nitrophenyl)-*N*,*N*-diethylacrylamide. HPLC Purity: 99.4%, 0.07% (Z)-isomer (72% yield).

### Example 6: Preparation of E-2-cyano-3-(3,4-dimethoxy-5-nitrophenyl)-N,N-diethylacrylamide from 3,4-dimethoxy-5-nitrobenzoic acid in one pot (compound of formula (II))

Under a nitrogen atmosphere, SOCl₂ (3.81 mL, 52.3 mmol) and DMF (0.17 mL, 2.2 mmol) were added to a suspension of 3,4-dimethoxy-5-nitrobenzoic acid (9.90 g, 43.6 mmol) in toluene (108 mL) at room temperature. The mixture was heated at 60 °C and stirred for 15 h. The organic solvent was reduced by distillation under reduced pressure to 50% of the starting volume. More toluene (2 x 50 mL) was added and finally eliminated to approximately 10% of the starting volume. The resulting slurry of 3,4-dimethoxy-5-nitrobenzoyl chloride was diluted with THF (46 mL).

60% NaH (3.66 g, 91.5 mmol) was added to a solution of 2-cyano-N,N-diethylacetamide (5.80 g, 41.4 mmol) in THF (140 mL) at -5 °C under a nitrogen atmosphere. The resulting suspension was stirred at -5 °C for 15 min and the THF solution of 3,4-dimethoxy-5-nitrobenzoyl chloride was added over 30 min and stirred for an additional 1 h at -5 °C. The reaction mixture was heated to 5 °C and quenched by the addition of AcOH (30 mL). The THF was eliminated under reduced pressure and the mixture was diluted with AcOH (50 mL). In a separate flask, 98% NaBH₄ (4.01 g, 103.7 mmol) was slowly added to glacial AcOH (80 mL) and stirred for 20 min at room temperature. The resulting suspension is added to the flask that contains 2-cyano-3-(3,4-dimethoxy-5-nitrophenyl)-*N,N-*diethyl-3-hydroxyacrylamide. The mixture was heated to 45 °C and stirred for 3.5 h. The solution was cooled to 30 °C and H₂O (170 mL) was added dropwise. The resulting suspension was stirred at 5 °C for 1 h. The solid was filtered off, washed with cold water and dried under reduced pressure to give 11.06 g of a solid with a 87% content in *E*-2-cyano-3-(3,4-dimethoxy-5-nitrophenyl)-*N*,*N*-diethylacrylamide (70% yield) and 13% of mineral oil. HPLC purity: 99.14%, (Z)-isomer: 0.11%.

### Example 7: Recrystallization of E-2-cyano-3-(3,4-dimethoxy-5-nitrophenyl)-N,N-diethylacrylamide

A suspension of 87% *E*-2-cyano-3-(3,4-dimethoxy-5-nitrophenyl)-*N*,*N-*diethylacrylamide (10.27 g), obtained as in Example 5, in 2-propanol (41 mL) was heated to 65 °C. The resulting solution was cooled to 55 °C over 30 min obtaining during this time a suspension of recrystallized product which was allowed to cool to room temperature over 1 h and stirred at 5 °C for 1 h more. The solid was collected by filtration, washed with cold 2-propanol and dried under reduced pressure to give 8.42 g (94%) of *E*-2-cyano-3-(3,4-dimethoxy-5-nitrophenyl)-N,N-diethylacrylamide. Off-white solid. Mp: 101.3-103.3°C. ¹H-RMN (400 MHz, CDCl₃) δ 7.87 (1 H, d, J = 1.9), 7.61 (1 H, d, J = 1.9), 7.56 (1 H, s), 4.04 (3H, s), 3.98 (3H, s), 3.49 (4H, broad q), 1.27 (6H, broad t). ¹³C-RMN (100.6 MHz, CDCl₃) δ 162.4, 154.2, 148.2, 145.3, 144.7, 127.7, 118.9, 115.7, 114.7, 108.5, 62.2, 56.6, 43.3, 40.8, 13.7, 12.5. HPLC purity: 99.63% , (Z)-isomer: 0.02%

### Example 8: Preparation of E-2-cyano-3-(3,4-dihydroxy-5-nitrophenyl)-N,N-diethylacrylamide using BBr₃ (compound of formula (I))

A 1.0 M solution of BBr₃ in CH₂Cl₂(1.62 ml, 1.62 mmol) was added dropwise to a solution of *E-*2-cyano-3-(3,4-dimethoxy-5-nitrophenyl)-*N,N-*diethylacrylamide (154 mg, 0.46 mmol) in CH₂Cl₂ (0.5 mL) at -15 °C under a nitrogen atmosphere. The resulting red suspension was stirred for 1 h at -15 °C and allowed to warm to room temperature overnight. The reaction was quenched by the addition of H₂O (4 mL) and stirred for 30 min. The aqueous phase was extracted with EtOAc (3 x 30 mL). The organic layers were combined, washed with saturated brine (20 mL) and dried (Na₂SO₄). The solvent was eliminated under reduced pressure to give 105 mg (74% yield) of *E*-2-cyano-3-(3,4-dihydroxy-5-nitrophenyl)-*N*,*N*-diethylacrylamide.

### Example 9: Preparation of E-2-cyano-3-(3,4-dihydroxy-5-nitrophenyl)-N,N-diethylacrylamide using AlCl₃ / pyridine (compound of formula (I))

AlCl₃ (371 mg, 2.78 mmol) and pyridine (0.54 mL, 6.69 mmol) were added portionwise to a solution of *E*-2-cyano-3-(3,4-dimethoxy-5-nitrophenyl)-*N*,*N-*diethylacrylamide (742 mg, 2.23 mmol) in N,N-dimethylformamide (6.4 mL) at 5 °C under a nitrogen atmosphere. The mixture was heated to 80 °C and stirred for 15 h. The resulting red solution was cooled to 10 °C and 35% aqueous HCl solution (1.0 mL) was added, followed by the addition of H₂O (7.4 mL) at 30 °C. Stirring was continued for 2 h at 5 °C and the precipitated solid was collected by filtration, washed with cold water and dried under reduced pressure to give 541 mg (80% yield) of *E-*2-cyano-3-(3,4-dihydroxy-5-nitrophenyl)-*N*,*N*-diethylacrylamide. HPLC purity: 96.0%, (Z)-isomer: 0.4%.

### Example 10: Preparation of E-2-cyano-3-(3,4-dihydroxy-5-nitrophenyl)-N,N-diethylacrylamide using AlCl₃ (compound of formula (I))

AlCl₃ (75.0 g, 0.56 mol) was added portionwise to a solution of *E-*2-cyano-3-(3,4-dimethoxy-5-nitrophenyl)-*N*,*N*-diethylacrylamide (150.0 g, 0.45 mmol) in N,N-dimethylformamide (1290 mL) at 5 °C under a nitrogen atmosphere. The mixture was heated to 80 °C and stirred for 15 h. The resulting red solution was cooled to 10 °C and 35% aqueous HCl solution (200 mL) was added, followed by the slow addition of H₂O (1500 mL) at 30 °C. The suspension was stirred at 5 °C for 2.5 h. The solid was filtered off, washed with cold water and dried under reduced pressure to give 126.3 g (92% yield) of *E*-2-cyano-3-(3,4-dihydroxy-5-nitrophenyl)-N,N-diethylacrylamide. Light yellow solid. ¹H-RMN (400 MHz, CDCl₃) δ 7.92 (1 H, d, J = 2.2), 7.73 (1 H, d, J = 2.2), 7.62 (1 H, s), 3.40 (4H, broad q), 1.14 (6H, broad t). Purity by HPLC: 99.7%, (Z)-isomer: 0.2%.

### Comparative Example 11. Demethylation process of E-2-cyano-3-(3,4-dimethoxy-5-nitrophenyl)-N,N-diethylacrylamide and 2-cyano-3-(3-methoxy-4-hydroxy-5-nitrophenyl)-N,N-diethyl-3-acrylamide

2-cyano-3-(3-methoxy-4-hydroxy-5-nitrophenyl)-N,N-diethyl-3-acrylamide was prepared by the method described in Example 2 of W02005/063693. Purity by HPLC of the crude monomethylated entacapone obtained: 90.4%; selectivity E/Z :75:25. Brown solid. The crude was recrystallized in methanol following the teachings of the mentioned patent application. Purity of the recrystallized product: 96.5%; selectivity E/Z: 93:7. Colour: intense yellow.

Crude and recrystallized 2-cyano-3-(3-methoxy-4-hydroxy-5-nitrophenyl)-N,N-diethyl-3-acrylamide was submitted to a demethylation reaction using AlCl₃ in the following reaction conditions:
Method A: AlCl₃ (1.25 eq) and pyridine (3.0 eq) were added to a solution of E-2-cyano-3-(3-methoxy-4-hydroxy-5-nitrophenyl)-N,N-diethylacrylamide (1.0 eq) in N,N-dimethylformamide (8.6 mL/g) at 5 °C under a nitrogen atmosphere. The mixture was heated to 80 °C- 90 °C and stirred for 15 h. The resulting red solution was cooled to 10-30 °C and 35% aqueous HCI solution (5 eq) and H₂O (10 mL/g) were added. Stirring was continued for 1-3 h at 5 °C and the precipitated solid was collected by filtration, washed with cold water and dried under reduced pressure to give E-2-cyano-3-(3,4-dihydroxy-5-nitrophenyl)-N,N-diethylacrylamide.
Method B: AlCl₃ (1.25 eq) was added portionwise to a solution of E-2-cyano-3-(3-methoxy-4-hydroxy-5-nitrophenyl)-N,N-diethylacrylamide (1.0 eq) in N,N-dimethylformamide (8.6 mL/g) at 5 °C under a nitrogen atmosphere. The mixture was heated to 80 °C and stirred for 16 h. The resulting red solution was cooled to 10 °C and 35% aqueous HCl solution (5 eq) was added, followed by addition of H₂O (10 mL/g) at 30 °C. The suspension was stirred at 5 °C for 2 h. The solid was filtered off, washed with cold water and dried under reduced pressure to give E-2-cyano-3-(3,4-dihydroxy-5-nitrophenyl)-N,N-diethylacrylamide.

Method A and B have also been carried out from *E*-2-cyano-3-(3,4-dimethoxy-5-nitrophenyl)-*N*,*N*-diethylacrylamide (see Examples 9 and 10).

The results are shown in the Table 1.

**Table 1:**

| **Starting material** | **Method** | **Reaction T (°C)** | **Yield** | **Purity HPLC final product (%)** | **E/Z final product** |
|---|---|---|---|---|---|
| Crude monomethylated entacapone | A | 90 | 61% | 56% | 83 : 17 |
| Crude monomethylated entacapone | B | 80 | 76% | 72% | 84:16 |
| Recrystallized monomethylated entacapone | A | 80 | 85% | 95.5% | 99.2:0.8 |
| Recrystallized monomethylated entacapone | B | 80 | 76% | 94.2% | 98.1:1.9 |
| Crude dimethylated entacapone (Example 9) | A | 80 | 80% | 96.0% | 99.6 : 0.4 |
| Crude dimethylated entacapone (Example 10) | B | 80 | 92% | 99.7% | 99.8:0.2 |

The results shown that when crude dimethylated entacapone is used as starting material a higher yield, higher purity and higher selectivity E/Z is achieved than when crude monomethylated entacapone is used as starting material. These results illustrate the fact that the use of dimethylated entacapone allows to obtain entacapone without need to carry out any additional purification process of the dimethylated entacapone or of the final entacapone obtained to remove the undesired (Z)-isomer or to eliminate the impurities that cause colouration.

This improvement observed by the use of dimethylated entacapone, in particular regarding purity and selectivity E/Z, is also observed even with regard to the monomethylated entacapone that has been recrystallized. Higher purity, and higher selectivity E/Z are achieved by using the dimethylated entacapone as starting material.

Furthermore, unlike what occurs when the monomethylated entacapone is used as starting material for the demethylation, when the dimetylated entacapone of the present invention is used, the demethylation reaction works better in the absence of pyridine since a significance improvement of the yield and of the purity is observed.

## Claims

1. A preparation process of entacapone of formula (I), comprising submitting the compound of formula (II) to a demethylation reaction.

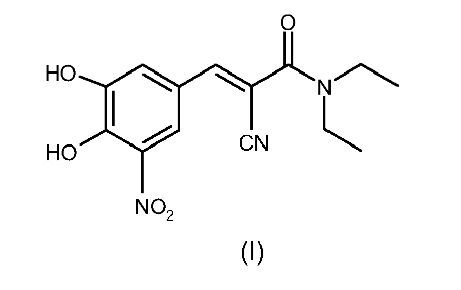

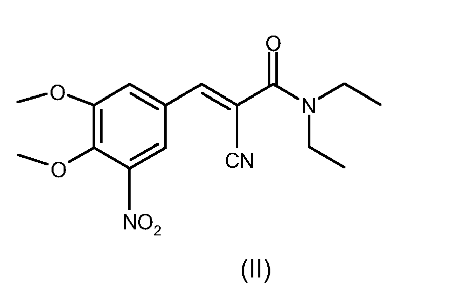

2. The preparation process according to claim 1, wherein the demethylation reaction is carried out using HBr or a Lewis acid.

3. The preparation process according to claim 2, wherein the Lewis acid is AlCl₃.

4. The preparation process according to any of the claims 1-3, wherein in a previous further step a compound of formula (III), 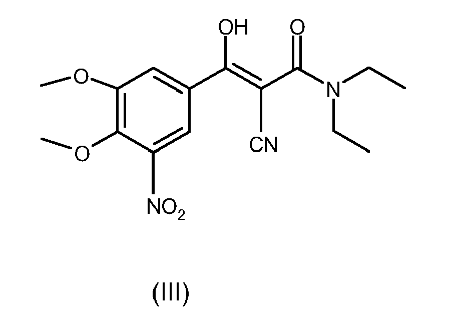 is reacted with a compound of formula MHB(OCOR)₃, in the presence of an appropriate solvent to give a compound of formula (II); wherein R is a radical selected from the group consisting of (C₁-C₄)-alkyl, phenyl, and CF₃; and M is a cation selected from the group consisting of sodium, potassium, and ((C₁-C₄)-alkyl)₄N.

5. The preparation process according to claim 4, wherein the compound of formula MHB(OCOR)₃ is NaHB(OAc)₃.

6. The preparation process according to any of the claims 4-5, wherein the solvent is acetic acid.

7. The preparation process according to any of the claims 4-6, wherein in a previous further step, an activated derivative of a compound of formula (VI) is reacted with a compound of formula (V) in the presence of a base, to give a compound of formula (III).

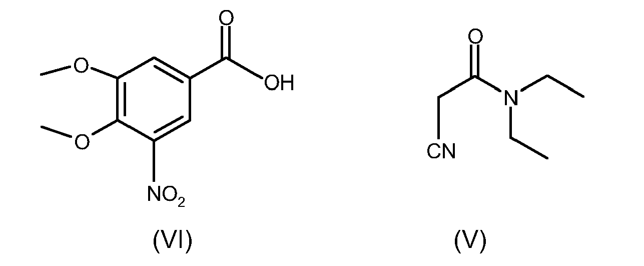

8. The preparation process according to claim 7, wherein the base is selected from the group consisting of sodium hydride, potassium tert-butoxide, magnesium ethoxide, and a mixture of triethylamine and magnesium chloride.

9. The preparation process according to any of the claims 6-8, wherein in a previous further step a compound of formula (VI) is reacted with an activating agent.

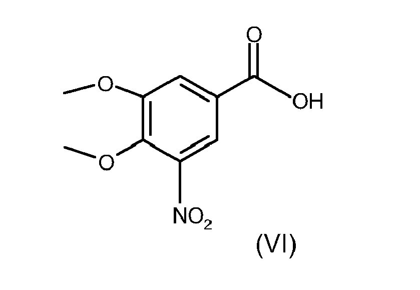

10. The preparation process according to claim 9, wherein the activating agent is selected from the group consisting of SOCl₂, PY₃, PY₅ wherein Y is Cl or Br, and oxalyl chloride, to give a compound of formula (IV) wherein X is Cl or Br.

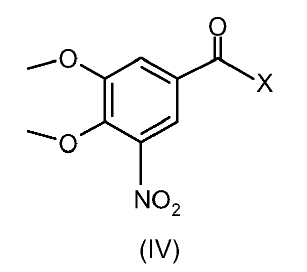

11. The preparation process according to claim 10, wherein the activating agent is SOCl₂ and in formula (IV) X is Cl.

12. The process according to any of the claims 1-11, wherein the steps needed for the preparation of the compound of formula (II) from the compound of formula (VI) are carried out in one pot, without isolating any intermediate compound.

13. A preparation process of a compound of formula (II), 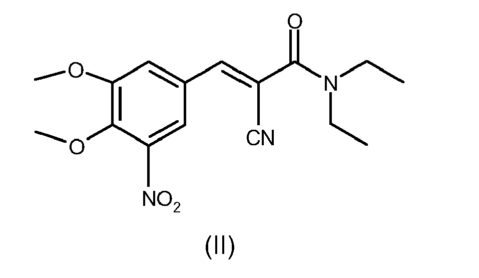 which comprises reacting a compound of formula (III) as defined in claim 4, with a compound of formula MHB(OCOR)₃, in the presence of an appropriate solvent to give a compound of formula (II); where R is a radical selected from the group consisting of (C₁-C₄)-alkyl, phenyl, and CF₃; and M is a cation selected from the group consisting of sodium, potassium, and ((C₁-C₄)-alkyl)₄N.

14. A preparation process of a compound of formula (III) 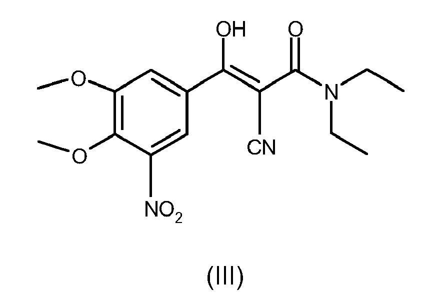 which comprises the following steps:
(a) reacting a compound of formula (VI) with an activating agent to give an activated derivative of the compound (VI), and 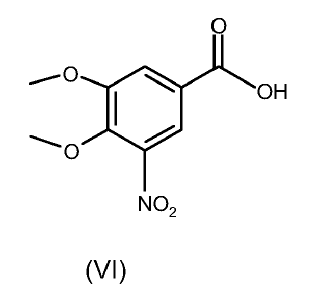
(b) reacting the activated derivative of compound of formula (VI) with a compound of formula (V), in the presence of a base, to give a compound of formula (III).

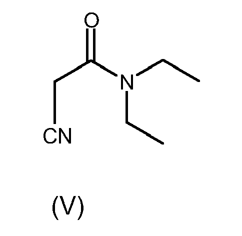

15. A compound of formula (II).

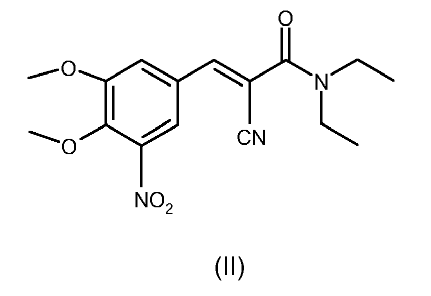

16. A compound of formula (III).
